# EUROPEAN PATENT SPECIFICATION

(11) **EP 1 325 745 B1**
(45) Date of publication and mention of the grant of the patent: **23.07.2008**
(21) Application number: 01974780.7
(22) Date of filing: 11.10.2001
(51) Int. Cl.: A61K 31/47, A61K 31/40, A61K 31/505, A61K 31/22, A61K 31/191, A61K 31/192, A61P 43/00, A61P 13/12, A61P 25/00, A61P 27/02, A61P 9/00, A61P 3/10, C07D 215/14, C07D 239/42, C07D 207/416

(54) **PREVENTIVES AND REMEDIES FOR COMPLICATIONS OF DIABETES**
WIRKSTOFFE ZUR VORBEUGUNG UND BEHANDLUNG VON KOMPLIKATIONEN IM ZUSAMMENHANG MIT DIABETES
MEDICAMENTS POUR LA PREVENTION OU LE TRAITEMENT DE COMPLICATIONS DU DIABETE

(30) Priority: 12.10.2000 JP 2000311960
(43) Date of publication of application: 09.07.2003
(73) Proprietor: Nissan Chemical Industries, Ltd., Chiyoda-ku, Tokyo 101-0054 (JP); Kowa Company. Ltd., Nagoya-shi, Aichi-ken 460-8625 (JP)
(72) Inventor: KITAHARA, Masaki, Shiraoka-machi, Minamisaitama (JP); SAITO, Yasushi, Chiba-shi, Chiba 260-0853 (JP); MORI, Seijiro, Chiba-shi, Chiba 260-0004 (JP); TAKEMOTO, Minoru, Akita-shi, Akita 010-0966 (JP); TAMAKI, Taro, Setagaya-ku, Tokyo 158-0094 (JP)
(74) Representative: HOFFMANN EITLE
(86) International application number: PCT/JP2001/008921
(87) International publication number: WO 2002/030425

(56) References cited:
- WO-A-00/53173
- WO-A-01/54694
- WO-A1-00/05213
- WO-A1-00/45818
- JP-A- 4 282 324
- MISRA ANOOP ET AL: "Simvastatin retards progression of diabetic retinopathy in hyperlipidemic patients: A double blind randomised control trial" DIABETES RESEARCH AND CLINICAL PRACTICE, vol. 50, no. Suppl. 1, September 2000 (2000-09), page S318, XP002328359 & 17TH INTERNATIONAL DIABETES FEDERATION CONGRESS ON DIABETES RESEARCH AND CLINICAL PRACTICE; MEXICO-CITY, MEXICO; NOVEMBER 05-10, 2000 ISSN: 0168-8227
- KIM SUNG IL ET AL: "Lovastatin inhibits transforming growth factor-beta1 expression in diabetic rat glomeruli and cultured rat mesangial cells" JOURNAL OF THE AMERICAN SOCIETY OF NEPHROLOGY, vol. 11, no. 1, January 2000 (2000-01), pages 80-87, XP002328360 ISSN: 1046-6673
- TAKEMOTO MINORU ET AL: "NK-104, a 3-hydroxy-3-methylglutaryl coenzyme A reductase inhibitor, reduces osteopontin expression by rat aortic smooth muscle cells" BRITISH JOURNAL OF PHARMACOLOGY, vol. 133, no. 1, May 2001 (2001-05), pages 83-88, XP002328362 ISSN: 0007-1188

## Description

### Technical Field

The present invention relates to the use of (+)-bis{(3R, 5S, 6E)-7-[2-cyclopropyl-4-(4-fluorophenyl)-3-quinolyl]-3,5-dihydroxy-6-heptenoic acid} calcium (hereinafter referred as pitavastatin calcium) or its lactonized form for the manufacture of a medicament for suppressing the expression of osteopontin in the kidney and blood vessels for the treatment of diabetic nephropathy, diabetic neuropathy, diabetic retinopathy or diabetic angiopathy.

### Background Art

Diabetes mellitus is known to lead to the diabetic complications such as diabetic nephropathy, diabetic neuropathy, diabetic retinopathy or diabetic angiopathy, and the strict control of the blood glucose may be required for their prevention and treatment thereof. The fibrosis and the calcification of the tissues are often observed in these complications. Under the high blood glucose condition, glycosylated proteins which are the modulators for cell function are produced, and the accumulation of sorbitol due to the activation of intracellular polyol pathway is observed, leading to the activation of intracellular protein kinase C (PKC) which results in abnormality of glomerular cells in the kidney, nerve cells or arterial endothelial cells, and induces the accumulation of extracellular matrices and the calcification.

The accelerated expression of extracellular matrices such as type IV collagen or fibronectin is well documented (Cagliero E. et al. : J. Clin. Invest., 82, 735-738 (1988), Haneda M. et al. : Diabetologia, 34, 198-200 (1991), Doi T. et al. : Proc. Natl. Acad. Sci. USA, 89, 2873-2877(1992)), but in recent days there are several papers reporting that the expression of osteopontin in the kidney and blood vessels markedly increases under diabetic condition and the expression of osteopontin thus accelerated may be in some ways related to diabetic nephropathy or diabetic angiopathy (Takemoto M. et al. : Arterioscler. Thromb. Vasc. Biol., 20, 624-628 (2000), Takemoto M. et al. : Ann. NY Acad. Sci., 902, 357-363 (2000)). From these findings, it is expected that the suppression of the expression of osteopontin as an extracellular matrices whose expression is accelerated in the kidney and arterial wall under the diabetic condition may be prophylactically effective on the onset or the aggravation of diabetic nephropathy or diabetic angiopathy.

At present, there is no pharmaceuticals discovered so far which control the essential quality of tissue lesions such as the expression and the production of extracellular matrices like osteopontin in order to prevent and/or treat diabetic complications such as diabetic nephropathy, diabetic neuropathy, diabetic retinopathy and diabetic angiopathy among others, and it is the high expectation to find pharmaceuticals having the excellent therapeutic effect for diabetic complications.

Hereupon, the compounds having inhibitory effect on HMG-CoA reductase activity were known to have the effects on the suppression of the cell proliferation, the suppression of cell adhesion, the suppression of intimal thickening and the prevention as well as the treatment of osteoporosis among others in addition to the main effect of inhibiting cholesterol biosynthesis. In addition, the suppression of the accumulation of fibronectin in the intimal lesion of the endothelial injury-induced neointima in the carotid artery had been reported (Kitahara M. et al. : Jpn. J. Pharmacol., 77, 117-128 (1998). However, there has been no report on the effect of the expression of osteopontin.

The object of the present invention is to provide the pharmaceuticals which can prevent and/or treat diabetic complications such as diabetic nephropathy, diabetic neuropathy, diabetic retinopathy and diabetic angiopathy among others by suppressing the expression of osteopontin in the kidney and blood vessels under the diabetic condition.

### Disclosure of invention

Knowing the present situation as described above, the inventors of the present invention administered a HMG-CoA reductase inhibitor, (+)-bis{(3R, 5S, 6E)-7-[2-cyclopropyl-4-(4-fluorophenyl)-3-quinolyl]-3,5-dihydroxy-6-heptenoic acid} calcium (hereafter referred as pitavastatin calcium) or its lactonized form, into streptozotocin (STZ)-induced diabetic rats and investigated the in detail effect on the expression of osteopontin mRNA in the kidney and blood vessels.
As a result pitavastatin calcium or a lactonized form thereof has shown the remarkable effect on the suppression of osteopontin mRNA expression, so the effectiveness of this compound on the prevention and/or treatment of diabetic complications was discovered and the present invention had been completed.
The present invention therefore provides the use of pitavastatin or a lactonized form thereof as a prophylactic and/or therapeutic agent for suppressing the expression of osteopontin in the kidney and blood vessels for the treatment of diabetic complications.

### Brief Description of Drawings

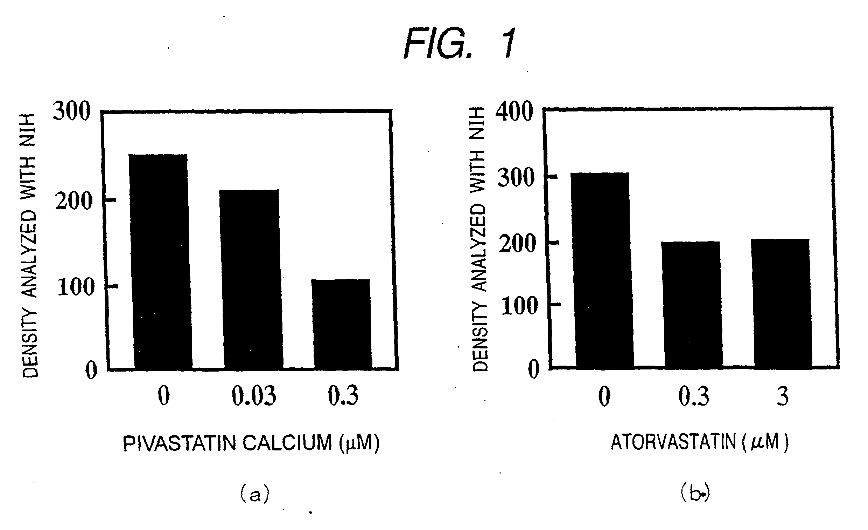

Figure 1(a) shows the effect of pitavastatin calcium on the secretion of osteopontin protein to the conditioned culture medium from aortic smooth muscle cells of rats cultured under the normal concentration of glucose, whereas Figure 1 (b) shows the effect of Atorvastatin on the secretion of osteopontin protein into the conditioned culture medium from aortic smooth muscle cells of rats cultured under the normal concentration of glucose.

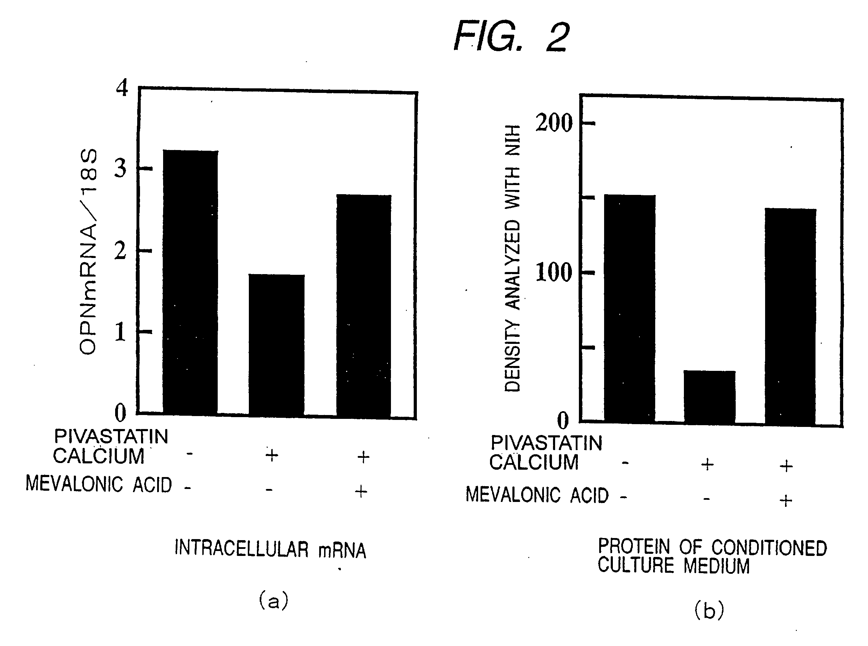

Figure 2(a) shows the influence of the addition of mevalonic acid on the suppressive effect of pitavastatin calcium on the expression of intracellular osteopontin mRNA in aortic smooth muscle cells of rats cultured under the normal concentration of glucose, whereas Figure 2(b) shows the influence of the addition of mevalonic acid on the suppressive effect of pitavastatin calcium on the secretion of osteopontin protein to the conditioned culture medium from aortic smooth muscle cells of rats cultured under the normal concentration of glucose.

### Best Mode for Carrying Out the Invention

Followings are the detailed description of the present invention.

Pitavastatin calcium or a lactonized form thereof has been known as the compound having the inhibitory effect on HMG-CoA reductase activity, but whether these compounds have any effect on the suppression of osteopontin expression, thereby useful as the pharmaceuticals in the treatment of diabetic complications or not has been elucidated so far.

Pitavastatin calcium is described in Japanese Patent No. 2,569,746, European Patent No. 304,063 or U.S. Patent No. 5,856,336.

Pitavastatin calcium suppresses at a statistically significant level the expression of osteopontin gene in the kidney and blood vessels of STZ-induced diabetic rats as well as the expression of osteopontin gene in the cultured vascular smooth muscle cells of rats as shown in the examples below. Therefore, pitavastatin calcium and a lactonized form thereof is useful for the prevention and/or the treatment of diabetic complications such as diabetic nephropathy, diabetic neuropathy, diabetic retinopathy, and diabetic angiopathy among others through the suppression of osteopontin expression. The use of the compound of the present invention makes it possible not only to prevent and to treat diabetic complications brought about with accelerated expression of osteopontin in diabetic patients, but also to exploit the possibilities of the new experimental systems as well as the new screenings for pharmaceuticals among other advantages.

The administration forms in using the compounds of the present invention as the pharmaceutical are, for example, oral administration forms such as tablet, capsule, granule, powder or syrup among others as well as parenteral administration forms such as intravenous injection, intramuscular injection, transdermal absorption, suppository, inhalation, ophthalmic solutions or collunarium among others. In addition, the active ingredient by itself can be used in order to produce the pharmaceutical preparations in these various forms, or any excipients, binders, fillers, disintegrators, surfactants, glossers, dispersion agents, buffers, preservatives, flavors, perfumes, coating agents, carriers, and diluents among others can appropriately be compounded therein.

Preferred form is the oral administration form among them, and the pH of the preparation is preferably adjusted in consideration for the stability of the active ingredient according to the methods described in Japanese Patent Application Laid-open No. Hei 2-6406, Japanese Patent No. 2,774,037, and WO97/23200.

The dose for the medical use of the present invention can be varied depending on the weight, age, gender as well as the symptoms of the patients, but 0.01 to 100mg per day, and especially 0.1 to 10mg per day of the compound shown in the formula (1) above is preferably administered in the form of once a day or twice a day for the adult in general.

### Examples

The usefulness of the present invention is described by referring to the following examples, but the invention is not limited to the examples described herewith.

### Example 1 The suppression of the expression of osteopontin mRNA in the kidney and blood vessels of streptozotocin (STZ)-induced diabetic rats

The effects of pitavastatin calcium on the expression of osteopontin mRNA in the kidney and blood vessels of STZ-induced diabetic rats were investigated according to the method described below.

Namely, 35mg per kg of body weight of STZ dissolved in the concentration of 50mg/mL of physiological saline was injected into the tail vein of male Wistar rats (body weight : about 300g), and the animals were orally administered 1mL/kg of body weight of 0.5% carboxymethylcellulose solution containing the test drug (pitavastatin calcium) in the concentration of 3mg/mL with a gastric sonde. Thereafter the oral administration was performed once a day by same volume and at fixed time during the experiment. The same volume of only 0.5% carboxymethylcellulose was given by the forced oral administration for the control group. The venous blood was withdrawn from the tail vein on the second day of the experiment and the presence of 200mg/dL or above of the blood sugar level was confirmed.

24 hours after 7 days administration, the blood was withdrawn under the ether anesthesia, and kidneys and thoracic aorta were isolated. The predetermined amount of the tissue piece in ISOGEN (Wako Pure Chemicals K.K.) was homogenized in a polytron homogenizer and the total RNA was extracted. The total RNA thus obtained was precipitated by using isopropanol. The precipitate was washed with 70% ice cold ethanol and stored at -80°C in 70% ethanol.

Osteopontin mRNA in the total RNA obtained was detected by the conventional Northern blotting method. That is to say that the total RNA precipitated with 70% ethanol was subjected to centrifugation at 15000 rpm, the precipitate was dried at room temperature after decanting the supernatant and dissolved in a small amount of TE buffer (10mM Tris-HCI buffer-1mM EDTA solution). 10µL out of the solution thus obtained was diluted with 990µL of TE buffer solution, and the amount of RNA was calculated by measuring the absorbance at 260nm ultraviolet light of the solution. 40% solution of deionized glyoxal (3.5µL), 0.1M of NaHPO₄ buffer solution (2.4µL) and dimethylsulfoxide (11.8µL) were added to the predetermined amount (10 or 20 µg) of the total RNA (final volume : 6µL), heated at 50°C for 1 hour and the total RNA was denatured. 6.3µL of 10mM sodium phosphate buffer solution (pH : 6.8) containing 50% glycerol and 0.4% bromophenol blue was added to the solution after the solution was cooled to the room temperature, and then RNA was subjected to electrophoresis using 1.5% agarose gel. RNA was blotted from agarose to nylon membrane in a conventional fashion by using 20X saline-sodium citrate (SSC). The blotted nylon membrane was washed with 2X SSC, and RNA was fixed on the nylon membrane by heating it to 80°C in vacuo. DNA fragment encoding osteopontin was digested from pCRIIrOP vector with *Eco* R1 endonuclease and purified with Probe Quant^{™}G-50 Micro Columns (Amersham Pharmacia Biotech Co. Ltd.). DNA fragment encoding Osteopontin thus obtained was hybridized for overnight together with the nylon membrane at 65°C with Rediprime^{™} II (Amersham Pharmacia Biotech Co. Ltd.) radioactive probe labeled with ³²P radioisotope. Radioisotope level of the probe bound to the nylon membrane was detected on the X-ray film and the density of the bands were analyzed according to NIH Image. 18S tRNA was used as the RNA internal standard and the amount of the expression was represented with the comparative intensities of the density of the bands. Osteopontin mRNA was similarly measured in the normal rat experiment.

The results of Example 1 are shown in Table 1.

In Table 1, OPN mRNA/18S represents the ratio of the density of osteopontin mRNA to the density of 18S tRNA based on the NIH Image Analysis and % inhibition represents that to the respective control groups. The values of OPNmRNA/18S are mean ± standard deviation.

**Table 1**

| | Kidney | | Aorta | |
|---|---|---|---|---|
| | OPNmRNA/18S | % Inhibition | OPNmRNA/18S | % Inhibition |
| Healthy control | 1.343±0.462 | | 2.400±1.345 | |
| | | | | |
| Healthy with drug administration | 1.667±0.321 | -24.1 | 2.433±0.929 | -1.4 |
| Diabetics control | 3.233±0.115 | | 3.200±0.361 | |
| | | | | |
| Diabetics with drug administration | 1.933±0.874* | 40.2 | 1.300±0.781** | 59.4 |

| | | | | |
|---|---|---|---|---|
| * : significantly different from the control; p=0.016 ** : significantly different from the control; p=0.036 OPN : osteopontin | | | | |

The ratio of osteopontin mRNA in the kidney and the aorta to 18S tRNA increased from 1.343 to 3.233 and 2.400 to 3.200 respectively in streptozotocin-induced diabetic rats. Pitavastatin calcium did not influence the expression of osteopontin in the kidney and the aorta of healthy rats (1.667 and 2.433 respectively), but decreased with the statistical significance the amount of the expression of osteopontin mRNA in the kidney and the aorta of STZ-induced diabetic rats to 1.933 (inhibition rate : 40.2%) and to 1.300 (inhibition rate : 59.4%), respectively.

### Example 2 The suppression of the secretion of osteopontin protein in aortic smooth muscle cells of rats

The effect of pitavastatin calcium and atorvastatin on the secretion of osteopontin protein into the conditioned culture medium from aortic smooth muscle cells of rats cultured under the normal glucose concentration were measured according to the method described below.

At first, aortic smooth muscle cells of rats (5 to 10 passage culture) were seeded in a 6-well culture plate and the confluent cultures were attained by culturing in low glucose (1000mg/L) Dulbecco's modified Eagle's medium (DMEM) with 10% fetal bovine serum (FBS : BioWhittaker Co. Ltd.) under 5% CO₂ atmosphere at 37°C. Thereafter, the medium was replaced with the medium with the test drugs (pitavastatin calcium and atorvastatin) and the cells were cultured for 48 hours. After the medium was again replaced with 1.5mL of FBS-free medium per well, the cells were cultured for additional 48 hours and the conditioned media were collected. The equal amount of 0.14M NaCI in 50mM Tris hydrochloride buffer solution (pH 7.4) was added to the predetermined volume (0.5-1mL) of the conditioned medium, and then 50µL of anion exchange DEAE cellulose ; DE52 (Whatman Co. Ltd.) suspended at 50% concentration after swelling and equilibrated with the same buffer described above was added, stirred gently for 1 hour at 4°C, and osteopontin protein was absorbed on DE52.

After centrifugation, sedimented DE52 gels were washed several times with the same buffer, and 60µL of 0.2M Tris hydrochloride buffer solution (pH 6.8) containing 5% of 2-mercaptoethanol, 4% of SDS, 5mL of EDTA, 20% of glycerol and 0.01% of bromophenol blue was added, and heat-treated for 5 minutes at 95°C. After cooling to the room temperature, the suspension was centrifuged and the predetermined amount (30µL) of the supernatant was subjected to 10% SDS polyacrylamide gel electrophoresis. After electrophoresis, proteins were transferred on nitrocellulose membrane according to the conventional technique and the Western blotting according to the conventional method was conducted. Namely, the nitrocellulose membrane was shaken in TBS-T (Tris buffer-physiological saline solution containing 0.2% Tween-20) with 3% bovine serum albumin for over 1 hour and the membrane was subsequently exposed with the same buffer described above containing anti-osteopontin antibody (MP IIIB10₁ ; American Research Products Co. Ltd.) at 1/1000 dilution for 1 hour with shaking. After that, the membrane was shaken for 1 hour in the horseradish peroxidase bound anti-mouse IgG antibody solution diluted to 1/5000 with TBS-T containing 3% bovine serum albumin and then washed with TBS-T for several times. Chemiluminescences were detected on X-ray film using ECL^{™} (Amersham Pharmacia Biotech Co. Ltd.). The density of the bands were analyzed according to NIH Image.

The above measurements were carried out with the concentration of 0µM (control), 0.03µM and 0.3µM for pitavastatin calcium, and with the concentrations of 0µM (control), 0.3µM and 3µM for atorvastatin, respectively.

The results of Example 2 were shown in Figure 1.

The density of osteopontin protein measured with NIH Image Analysis for various concentration of pitavastatin calcium are shown in Figure 1(a), and the density of osteopontin protein measured with NIH Image Analysis for various concentration of atorvastatin are shown in Figure 1(b).

It is clear from Figure 1 that both pitavastatin calcium and atorvastatin inhibited with the statistical significance the amount of the secretion of osteopontin protein into the conditioned medium from the cultured aortic smooth muscle cells of rats.

### Example 3 The effect of mevalonic acid on the expression of osteopontin mRNA and the suppression of the secretion of osteopontin protein in aortic smooth muscle cells of rats

The effect of mevalonic acid on the suppression with pitavastatin calcium for the expression of osteopontin mRNA and the secretion of osteopontin protein in aortic smooth muscle cells of rats were measured according to the method described below.

Aortic smooth muscle cells of rats (5 to 10 passage culture) were seeded in a 6-well culture plate and the confluent cultures were attained by culturing in low glucose (1000mg/L) DMEM with 10% FBS under 5% CO₂ atmosphere at 37°C. Thereafter, the medium was replaced with the medium with pitavastatin calcium (8µ M) and/or mevalonic acid (100µM), and the cells were cultured for another 48 hours. The medium was again replaced with 1.5mL of FBS-free medium per well, and the cells were cultured further for 48 hours.

After the cultivation, the cells adhered to the culture plate were homogenized together with ISOGEN, RNA were extracted exactly as in Example 1, and the amount of osteopontin mRNA was analyzed by northern blotting method.

At the same time, the conditioned medium was collected, osteopontin protein was absorbed on DE52, subjected to electrophoresis exactly as in Example 2, and the amount of the secreted osteopontin protein was analyzed by western blotting method.

The above measurements were carried out in the three cases when pitavastatin calcium and mevalonic acid were not added, when only pitavastatin calcium was added, and when both pitavastatin calcium and mevalonic acid were added.

The results of Example 3 were shown in Figure 2.

Figure 2(a) shows the ratio of the density of osteopontin mRNA band to the density of 18S tRNA band according to NIH Image Analysis, and Figure 2(b) shows the density of osteopontin protein according to NIH Image Analysis for three conditions described above.

Although pitavastatin calcium suppresses both the expression of osteopontin mRNA and the secretion of osteopontin protein from cultured smooth muscle cells of rats, it is clear from Figure 2 that these suppressive effect with pitavastatin calcium disappear with the addition of mevalonic acid. From the fact, it is found that the addition of pitavastatin calcium suppresses the production of mevalonic acid, thereby suppressing the expression of osteopontin mRNA as well as the secretion of protein thereof in aortic smooth muscle cells.

### Industrial Applicability

Pitavastatin calcium shows the specific and effective inhibitory action against the accelerated expression of osteopontin in the kidney and the aorta afflicted with diabetic condition without affecting the expression of osteopontin under healthy condition, and markedly suppresses the biosynthesis of osteopontin in these organs in diabetes.

Therefore, pitavastatin calcium or a lactonized form thereof is especially useful as prophylaxis and/or treatment drug for suppressing the expression of osteopontin in the kidney and blood vessels for treating diabetic complications possibly brought about by the accelerated expression of osteopontin gene such as diabetic nephropathy, diabetic neuropathy, diabetic retinopathy and diabetic angiopathy among others.

## Claims

1. The use of (+)-bis{(3R, 5S, 6E)-7-[2-cyclopropyl-4-(4-fluorophenyl)-3-quinolyl]-3,5-dihydroxy-6-heptenoic acid} calcium or its lactonized form for the manufacture of a prophylactic and/or therapeutic medicament for suppressing the expression of osteopontin in the kidney and blood vessels for the treatment of diabetic nephropathy, diabetic neuropathy, diabetic retinopathy or diabetic angiopathy.

## Patentansprüche

1. Verwendung von (+)-Bis{(3R,5S,6E)-7-[2-cyclopropyl-4-(4-fluorphenyl)-3-chinolyl]-3,5-dihydroxy-6-heptensäure}calcium oder dessen lactonisierter Form zur Herstellung eines prophylaktischen und/oder therapeutischen Medikaments zur Unterdrückung der Expression von Osteopontin in der Niere und Blutgefäßen zur Behandlung von diabetischer Nephropathie, diabetischer Neuropathie, diabetischer Retinopathie oder diabetischer Angiopathie.

## Revendications

1. Utilisation de l'acide (+)-bis{(3R, 5S, 6E)-7-[2-cyclopropyl-4-(4-fluorophényl)-3-quinolyl]-3,5-dihydroxy-6-hepténoïque} calcium ou de sa forme lactonisée pour la fabrication d'un médicament prophylactique et/ou thérapeutique destiné à supprimer l'expression de l'ostéopontine dans les vaisseaux sanguins et dans le rein pour le traitement de néphropathie diabétique, neuropathie diabétique, rétinopathie diabétique ou angiopathie diabétique.
